(19) Europäisches Patentamt / European Patent Office / Office européen des brevets

(11) EP 4 491 659 A1

(12) **EUROPEAN PATENT APPLICATION**
published in accordance with Art. 153(4) EPC

(43) Date of publication:
**15.01.2025 Bulletin 2025/03**

(21) Application number: 23779640.4

(22) Date of filing: **15.03.2023**

(51) International Patent Classification (IPC):
*C08J 3/14* (2006.01)      *A61F 13/53* (2006.01)
*C08F 2/32* (2006.01)      *C08F 8/50* (2006.01)
*C08F 220/06* (2006.01)

(52) Cooperative Patent Classification (CPC):
**A61F 13/53; C08F 2/32; C08F 8/50; C08F 220/06;
C08J 3/14**

(86) International application number:
**PCT/JP2023/010167**

(87) International publication number:
**WO 2023/189622 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.03.2022   JP 2022056400**

(71) Applicant: **Sumitomo Seika Chemicals Co., Ltd.
Kako-gun, Hyogo 675-0145 (JP)**

(72) Inventor: **NISHIDA Moe
Himeji-shi, Hyogo 672-8076 (JP)**

(74) Representative: **Vossius & Partner
Patentanwälte Rechtsanwälte mbB
Siebertstrasse 3
81675 München (DE)**

(54) **WATER ABSORBENT RESIN PARTICLES, ABSORBER AND ABSORBENT ARTICLE**

(57)     Disclosed are water absorbent resin particles that contain a crosslinked polymer that has, as a monomer unit, at least one kind of ethylenically unsaturated monomer selected from the group consisting of (meth) acrylic acid and a salt thereof. The water absorbent resin particles have a centrifuge retention capacity of 30 g/g or more and a gel flow angle of 10° or more and 35° or less.

*Fig.8*

EP 4 491 659 A1

## Description

### Technical Field

The present disclosure relates to water absorbent resin particles, an absorber, and an absorbent article.

### Background Art

Absorbers containing water absorbent resin particles are widely used in the fields of sanitary materials such as paper diapers and sanitary products, agricultural and horticultural materials such as water retention agents and soil conditioners, and industrial materials such as water cutoff agents and condensation-preventing agents (for example, Patent Literature 1). In addition to the water absorbent resin particles, the absorber often contains a certain amount of pulp.

### Citation List

### Patent Literature

[Patent Literature 1] PCT International Publication No. WO2020/184389

### Summary of Invention

### Technical Problem

From the viewpoint of reducing the environmental impact and the like, it is sometimes required to reduce a pulp content in an absorber or to eliminate pulp from an absorber. However, in a case where the proportion of water absorbent resin particles is high in the absorber, the absorber is likely to move or deform due to external stress after liquid absorption. Therefore, the water absorption performance of the absorber tends to easily decrease to a low level as compared with the original level due to the movement or deformation of the absorber. For example, in a case where an absorber that has absorbed a liquid moves or deforms due to external stress, the amount of the liquid that returns onto the surface of the absorber in a case where a load is applied to the absorber (hereinafter, may be referred to as the "amount of re-wet") may increase significantly.

An aspect of the present disclosure relates to water absorbent resin particles that can suppress an increase in the amount of re-wet when an absorber has moved or deformed after liquid absorption, even in the case of an absorber containing water absorbent resin particles at a high proportion.

### Solution to Problem

An aspect of the present disclosure generally includes the following solutions.

[1] Water absorbent resin particles comprising:
a crosslinked polymer comprising, as a monomer unit, at least one kind of ethylenically unsaturated monomer selected from the group consisting of (meth)acrylic acid and salts thereof. The water absorbent resin particles have a centrifuge retention capacity of 30 g/g or more and a gel flow angle of 10° or more and 35° or less. The gel flow angle is measured in an environment of a temperature of 25°C ± 2°C and a relative humidity of 50% ± 10%, according to a method which includes:

disposing an acrylic plate that has a rectangular working surface having a long side and a short side so that the working surface is horizontal,
placing a guide frame that has a thickness of 2 cm and has a rectangular opening having a long side of 12 cm and a short side of 8 cm on the working surface so that the short side of the opening is parallel to the short side of the working surface,
forming, in the opening, a film-shaped absorber consisting of 3.00 ± 0.01 g of a powder of the water absorbent resin particles,
subjecting the absorber in the opening to first charge with 40 mL of saline at a speed of 8 m/sec, and 5 minutes after start of the first charge of the saline, subjecting the absorber to second charge with 40 mL of saline at a speed of 8 m/sec, thereby forming, on the working surface in the opening, a gel film having a rectangular main surface having a long side of 12 cm and a short side of 8 cm parallel to the short side of the working surface,
5 minutes after start of the second charge of the saline, placing, on the gel film, a plate-shaped weight that weighs 222 g and has a rectangular main surface having a long side of 11.9 cm and a short side of 7.9 cm,
1 minute after the weight is placed on the gel film, removing the weight and the guide frame,
raising an end part of the acrylic plate on one side of the short side of the working surface, thereby tilting the acrylic plate and the gel film over a period of 5 seconds until an angle θ between the working surface and a horizontal plane is 10°,
12 minutes after the start of the first charge of the saline, placing the weight on the working surface on an upper side of the tilted gel film so that an entire end surface of the weight on a side of the short side comes into contact with an end surface of the gel film on a side of the short side,
every 2 minutes after the weight is placed on the working surface, further raising the end part of the acrylic plate so that the angle θ increases by 5° over a period of 5 seconds, and
recording, as a gel flow angle, an angle θ at a time when the weight moves on the working surface by 10 cm downward along a longitudinal direction of the working surface while pushing the gel film.

[2] The water absorbent resin particles according to [1], wherein the water absorbent resin particles have a median particle diameter of 200 μm or more and 600 μm or less.

[3] The water absorbent resin particles according to [1], wherein the water absorbent resin particles have an average value of an aspect ratio of 1.0 or more and 1.5 or less.

[4] The water absorbent resin particles according to [2], wherein the water absorbent resin particles have an average value of an aspect ratio of 1.0 or more and 1.5 or less.

[5] An absorber comprising:
the water absorbent resin particles according to any one of [1] to [4].

[6] The absorber according to [5], wherein a content of the water absorbent resin particles is 90% by mass or more and 100% by mass or less based on a mass of the absorber.

[7] An absorbent article comprising:
the absorber according to [5] or [6].

**Advantageous Effects of Invention**

It is possible to suppress an increase in an amount of re-wet when an absorber has moved or deformed after liquid absorption, even in the case of an absorber containing water absorbent resin particles at a high proportion.

**Brief Description of Drawings**

FIG. 1 shows schematic views showing a method of measuring a gel flow angle.
FIG. 2 shows schematic views showing the method of measuring a gel flow angle.
FIG. 3 shows schematic views showing the method of measuring a gel flow angle.
FIG. 4 shows a schematic view showing the method of measuring a gel flow angle.
FIG. 5 shows schematic views showing the method of measuring a gel flow angle.
FIG. 6 shows a schematic view showing the method of measuring a gel flow angle.
FIG. 7 is a plan view showing one example of a guide frame that is used for measuring a gel flow angle.
FIG. 8 is a cross-sectional view showing one example of an absorbent article having an absorber.
FIG. 9 is a schematic view showing a method of measuring the absorption against pressure.

**Description of Embodiments**

The present invention is not limited to the following examples. In the present specification, "(meth)acrylic" means both acrylic and methacrylic. Similarly, "acrylate" and "methacrylate" are also written as "(meth)acrylate". The same applies to other similar terms. The term "(poly)" means both a case where the prefix "(poly)" is present and a case where the prefix "(poly)" is not present. In numerical value ranges described stepwise in the present specification, any combination is possible between an upper limit value or lower limit value of a numerical value range of a certain stage and an upper limit value or lower limit value of a numerical value range of another stage. With regard to a numerical value range described in the present specification, the upper limit value or lower limit value of the numerical value range may be replaced with the values disclosed in Examples. The term "watersoluble" refers to a solubility of 5% by mass or more in water at 25°C. The materials exemplified in the present specification may be used alone or two or more kinds thereof may be used in combination. The term "saline" refers to an aqueous solution of 0.9% by weight of sodium chloride.

One example of the water absorbent resin particles includes particles containing a crosslinked polymer that has, as a monomer unit, at least one kind of ethylenically unsaturated monomer selected from the group consisting of (meth) acrylic acid and a salt thereof. The total proportion of a monomer unit derived from (meth)acrylic acid and a monomer unit derived from a (meth)acrylic acid salt may be 70% by mole or more and 100% by mole or less, 80% by mole or more and 100% by mole or less, or 90% by mole or more and 100% by mole or less, based on the total amount of monomer units in the crosslinked polymer.

The (meth)acrylic acid salt may be an alkali metal salt, an ammonium salt, or a combination thereof. The alkali metal salt may be a sodium salt, a potassium salt, or a combination thereof. The proportion (degree of neutralization) of the monomer unit derived from the (meth)acrylic acid salt may be 10% by mole or more, 50% by mole or more, or 60% by mole or more, and may be 100% by mole or less, 90% by mole or less, or 80% by mole or less, based on the total amount of the monomer unit derived from (meth)acrylic acid and the monomer unit derived from a (meth)acrylic acid salt.

The crosslinked polymer constituting the water absorbent resin particles may further contain, as a monomer unit, another ethylenically unsaturated monomer different from (meth)acrylic acid and a salt thereof. The other ethylenically unsaturated monomer can include, for example, at least one compound selected from the group consisting of 2-(meth) acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide.

The crosslinked polymer can include a crosslinking structure due to self-crosslinking of an ethylenically unsaturated monomer, a crosslinking structure formed by a reaction of a crosslinking agent with an ethylenically unsaturated monomer, or both thereof. The crosslinked polymer may include a crosslinking structure formed by an internal crosslinking agent that reacts with an ethylenically unsaturated monomer during the polymerization of the ethylenically unsaturated monomer, a surface crosslinking agent that reacts, in a polymer particle containing an ethylenically unsaturated monomer, with a polymer mainly in a surface layer portion of the polymer particle, or both thereof. The polymer chains that constitute the crosslinked polymer may be entangled with each other to form a pseudo-crosslinking structure.

The water absorbent resin particles may have a centrifuge retention capacity (hereinafter, may be referred to as "CRC") of 30 g/g or more. A large CRC contributes to a decrease in the absolute value of the amount of re-wet from an absorber containing water absorbent resin particles at a high proportion. The CRC may be 31 g/g or more, 32 g/g or more, 33 g/g or more, 34 g/g or more, 35 g/g or more, 36 g/g or more, 37 g/g or more, 38 g/g or more, 39 g/g or more, 40 g/g or more, 41 g/g or more, 42 g/g or more, 43 g/g or more, 44 g/g or more, 45 g/g or more, 46 g/g or more, 47 g/g or more, or 48 g/g or more. The CRC may be 65 g/g or less, 60 g/g or less, 55 g/g or less, or 50 g/g or less. The CRC may be 30 g/g or more and 65 g/g or less. The CRC can be adjusted, for example, based on the degree of crosslinking in the crosslinked polymer. In a case where the amount of the internal crosslinking agent that is used in the polymerization reaction is small, the CRC tends to be large.

In the present specification, the CRC is a value that is measured in an environment of a temperature of 25°C $\pm$ 2°C and a relative humidity of 50% $\pm$ 10%. A method of measuring the CRC includes;

immersing a nonwoven fabric bag that has a rectangular main surface of 60 mm $\times$ 85 mm and is accommodating polymer particles (or water absorbent resin particles) having a mass Mc (g), in saline having a temperature of 25°C $\pm$ 2°C for 30 minutes, thereby swelling the polymer particles to form a gel,
applying a centrifugal force of 250 G for 3 minutes with a centrifuge to dehydrate the gel in the nonwoven fabric bag, measuring a mass Ma (g) of the gel and nonwoven fabric bag after the dehydration,
immersing the nonwoven fabric bag that is not accommodating polymer particles, in the saline for 30 minutes, and then applying a centrifugal force of 250 G for 3 minutes with a centrifuge to dehydrate the immersed nonwoven fabric bag and measuring a mass Mb (g) of the nonwoven fabric bag after the dehydration, and
calculating the CRC according to the following expression:

$$CRC\ [g/g] = \{(Ma - Mb) - Mc\}/Mc.$$

Mc is 0.2 $\pm$ 0.002 g. While the nonwoven fabric bag accommodating the polymer particles is immersed in the saline, the saline may be stirred as necessary so that the polymer particles absorb the maximum amount of water.

The water absorbent resin particles may have a gel flow angle of 10° or more and 35° or less. The gel flow angle is a value that reflects the flowability of the gel that is formed by the water absorbent resin particles absorbing water, and a large value of the gel flow angle means that the flowability of the gel is relatively small. A gel having low flowability is resistant to a weak external stress, and it is likely to move or deform significantly in a case of being subjected to a strong external stress. In a case where the gel moves or deforms significantly, it is considered that an unequal distribution of the gel in the absorber occurs, which results in an increase in the amount of re-wet. A gel having such a moderate flowability in which a gel flow angle of 10° or more and 35° or less is exhibited moves gradually due to a relatively weak external stress. Therefore, it is conceivable that the unequal distribution is less likely to occur, and the amount of re-wet is less likely to increase. From the same viewpoint as above, the gel flow angle may be 15° or more, or 20° or more, and it may be 30° or less, or 25° or less.

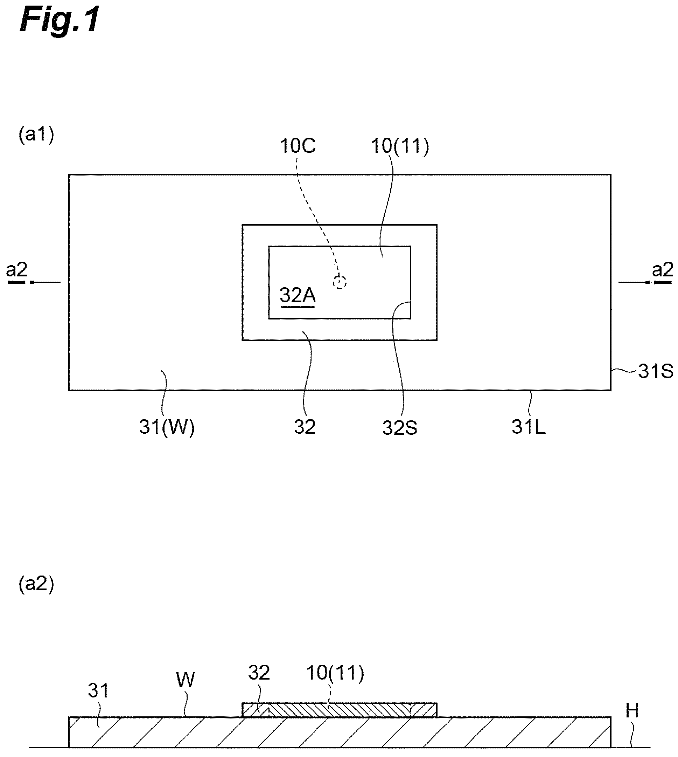

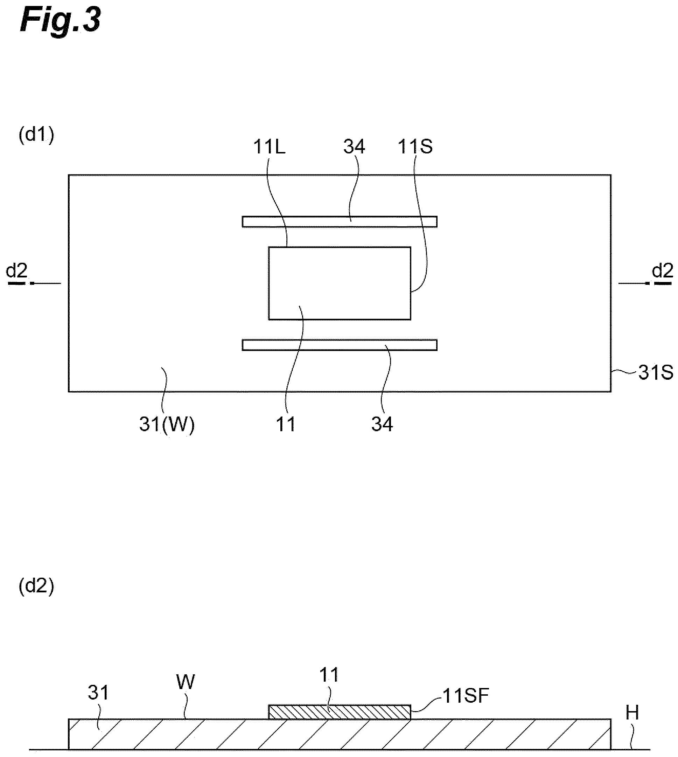

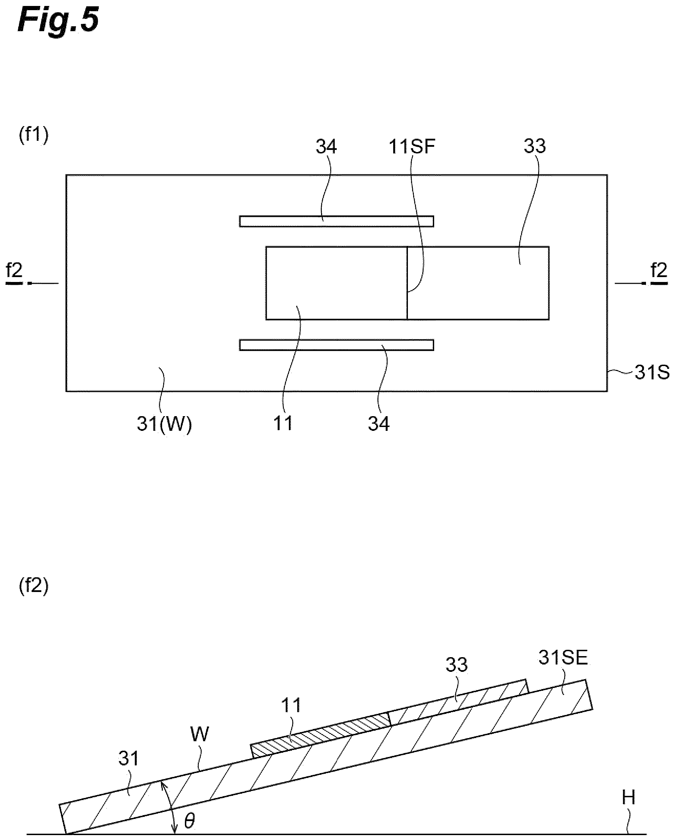

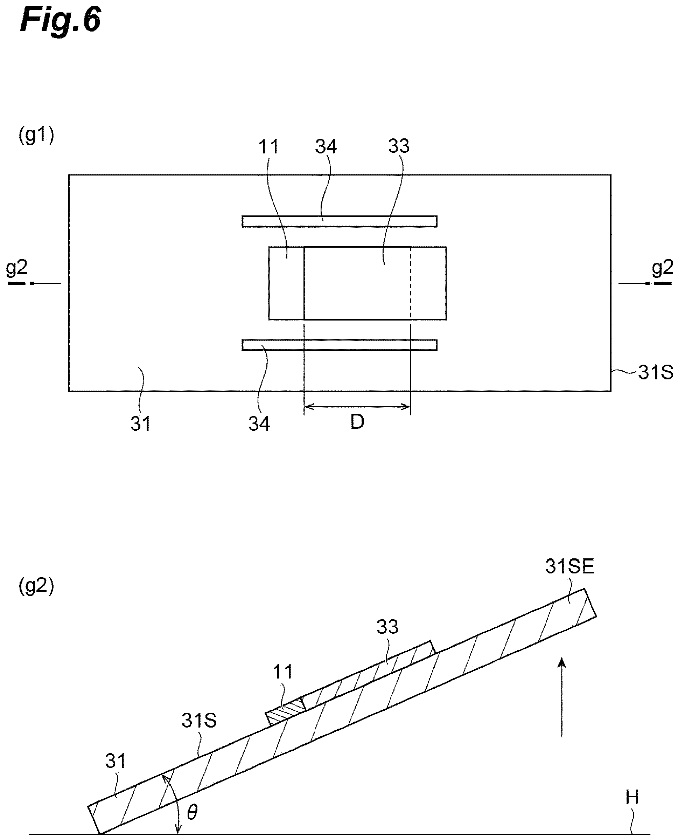

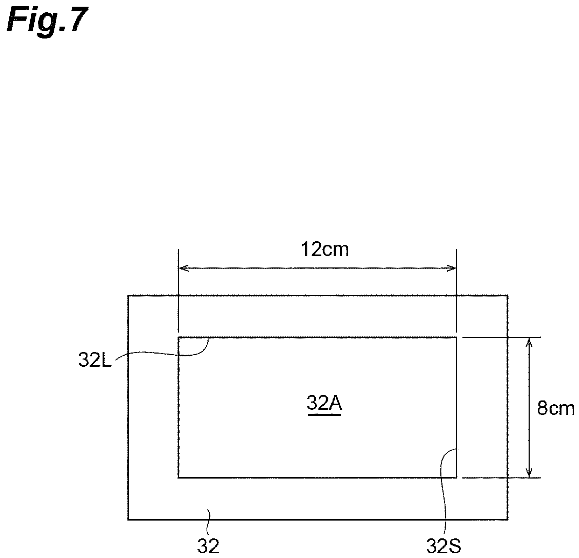

FIGS. 1 to 6 are schematic views showing a method of measuring the gel flow angle of water absorbent resin particles. FIG. 7 is a plan view showing one example of a guide frame that is used in this method. In FIG. 1, (a1) is a plan view, and (a2) is an end view taken along a line a2-a2 in (a1). In FIG. 3, (d1) is a plan view, and (d2) is an end view taken along a line d2-d2 in (d1). In FIG. 5, (f1) is a plan view, and (f2) is an end view taken along a line f2-f2 in (f1). In FIG. 6, (g1) is a plan view, and (g2) is an end view taken along a line g2-g2 in (g1).

In the method shown in FIG. 1 to FIG. 6, the measurement is carried out by a method which includes;

disposing, on a horizontal flat surface H, an acrylic plate 31 that has a rectangular working surface W having a long side 31L and a short side 31S so that the working surface W is horizontal,

placing a guide frame 32 that has a thickness of 2 cm and has a rectangular opening 32A having a long side 32L of 12 cm and a short side 32S of 8 cm on the working surface W so that the short side 32S of the opening 32A is parallel to the short side 31S of the working surface W,

forming, in the opening 32A, a film-shaped absorber 10 consisting of 3.00 ± 0.01 g of a powder of the water absorbent resin particles, subjecting a central part 10C of the absorber 10 in the opening 32A to a first charge with 40 mL of saline at a speed of 8 m/sec, and 5 minutes after start of the first charge of the saline,

subjecting the absorber 10 to a second charge with 40 mL of saline at a speed of 8 m/sec, thereby forming, on the working surface W in the opening 32A, a gel film 11 having a rectangular main surface having a long side of 12 cm and a short side of 8 cm parallel to the short side of the working surface,

5 minutes after start of the second charge of the saline, placing, on the gel film 11, a plate-shaped weight 33 that weighs 222 g and has a rectangular main surface having a long side of 11.9 cm and a short side of 7.9 cm,

1 minute after the weight 33 is placed on the gel film 11, removing the weight 33 and the guide frame 32,

raising an end part 31SE of the acrylic plate 31 on one side of the short side 31S of the working surface W, thereby tilting the acrylic plate 31 and the gel film 11 over a period of 5 seconds until an angle θ between the working surface W and a horizontal plane (flat surface H) is 10°,

12 minutes after the start of the first charge of the saline, placing the weight 33 on the working surface W on an upper side of the tilted gel film 11 so that an entire end surface of the weight 33 on a side of the short side comes into contact with an end surface 11SF of the gel film 11 on a side of the short side 11S,

every 2 minutes after the weight 33 is placed on the working surface W, further raising the end part 31 SE of the acrylic plate 31 so that the angle θ increases by 5° over a period of 5 seconds, and

recording, as a gel flow angle, an angle θ at a time when the weight 33 moves on the working surface W by 10 cm downward along a longitudinal direction of the working surface W while pushing the gel film 11. The gel flow angle is measured in an environment of a temperature of 25°C ± 2°C and a relative humidity of 50% ± 10%. The temperatures of the water absorbent resin particles, the saline, and other members that are used in the measurement are also 25°C ± 2°C.

The acrylic plate 31 is a plate-shaped molded body made of an acrylic resin, which has a flat main surface as the working surface W. The working surface W has a size sufficient for disposing the guide frame 32 and the weight 33. For example, the length of the long side 31L may be 40 cm, and the length of the short side 31S may be 8 cm.

The guide frame 32 can be, for example, a molded body made of an acrylic resin. The guide frame 32 is usually placed in the central part of the working surface W.

In a case where a predetermined amount of a powder of the water absorbent resin particles is uniformly scattered on the working surface W in the opening 32A of the guide frame 32, the film-shaped absorber 10 is formed in the opening 32A. 40 mL of the saline is charged twice toward the central part 10C of the absorber 10. The saline is charged using, for example, a burette. The time taken from the start of the first charge of the saline to the second charge of the saline is 5 minutes. The absorber 10 absorbs the saline forms the gel film 11 that has a rectangular main surface having a long side and a short side and corresponds to the opening 32A is formed through absorbing the saline.

5 minutes after the start of the second charge of the saline, a plate-shaped weight 33 that weighs 222 g and has a rectangular main surface having a long side of 11.9 cm and a short side of 7.9 cm is gently placed on the gel film 11. The weight 33 can be, for example, a molded body made of an acrylic resin. The weight 33 has a main surface that is slightly smaller than the opening 32A, and thus most of the opening 32A is blocked by the weight 33.

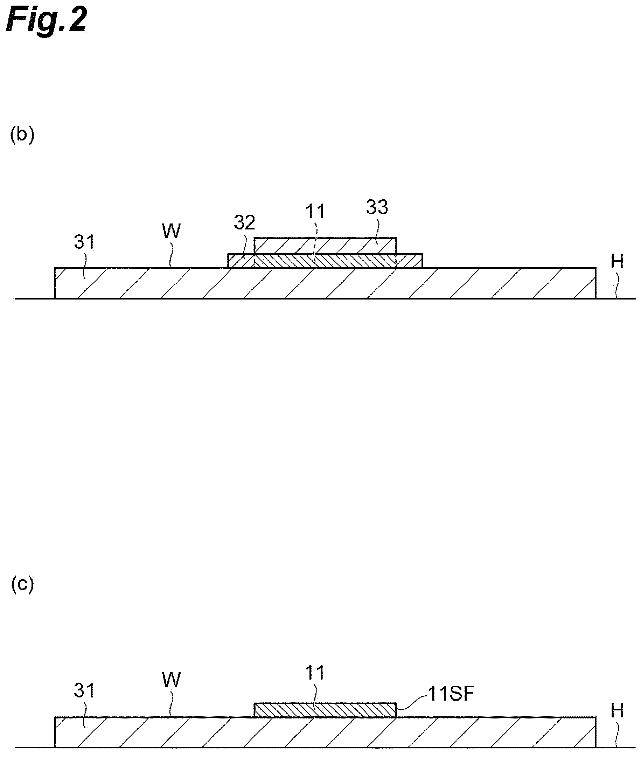

1 minute after the weight 33 is placed on the gel film 11, the weight 33 and the guide frame 32 are gently removed so that the gel film 11 does not move or deform. At this time, as shown in FIG. 2(b), the gel film 11 formed on the working surface W is in a state of being entirely exposed, including the end surface 11SF on the side of the short side.

Subsequently, as shown in FIG. 3, a wall 34 may be provided on the working surface W, where the wall 34 is along the long side 11L of the gel film 11. The distance between the wall 34 and the end surface of the gel film 11 on the side of the long side 11L side may be, for example, 1 cm. Two walls 34 having a length larger than the long side 11L of the gel film 11 may be disposed so that the entire gel film 11 is located therebetween.

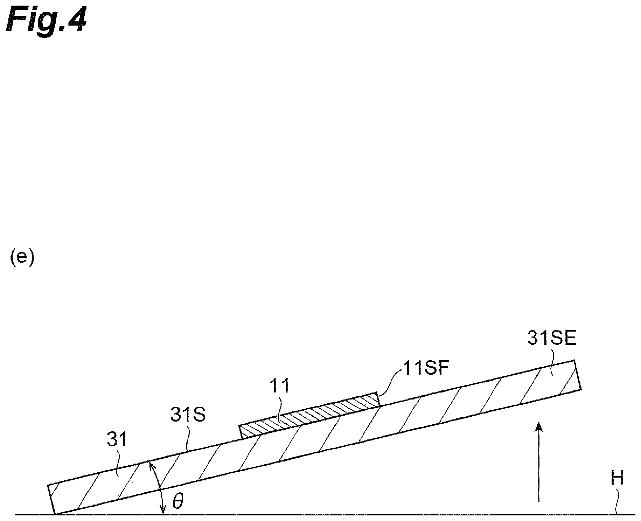

As shown in FIG. 4, the end part 31SE of the acrylic plate 31 on one side of the short side 31S of the working surface W is raised, whereby the acrylic plate 31 and the gel film 11 are tilted until an angle θ between the working surface W and a horizontal plane (flat surface H) is 10°. The end part 31SE of the acrylic plate 31 is raised at a substantially constant speed over a period of 5 seconds until the angle θ reaches 10°, in a state where an end part of the acrylic plate 31 on a side opposite to the end part 31SE is in contact with the flat surface H. In a state where the angle θ is 10°, the acrylic plate 31 is temporarily fixed.

12 minutes after the start of the first charge of the saline, as shown in FIG. 5, the weight 33 is gently placed on the working surface W on the upper side of the tilted gel film 11 so that the entire end surface of the weight 33 on the side of the short side, where the end surface is perpendicular to the working surface W comes into contact with the end surface 11SF of the gel film 11 on the side of the short side, where the end surface is perpendicular to the working surface W. At this time, the weight 33 placed on the working surface W may move downwards on the working surface W along the longitudinal direction of the working surface W while pushing the gel film 11.

Every 2 minutes after the weight 33 is placed on the working surface W, the end part 31SE of the acrylic plate 31 is further raised so that the angle θ increases by 5° over a period of 5 seconds (FIG. 6). As the angle θ increases, a movement distance D of the weight 33 increases. The movement distance D is the distance moved by the end surface of the weight 33 in contact with the gel film 11 in the longitudinal direction of the working surface W from the initial position. In a case where the angle θ is increased stepwise by 5°, an angle θ at the time when the moving distance D first reaches 10 cm is recorded as the gel flow angle. In a case where the moving distance D reaches 10 cm at the time when the angle θ is 10°, the gel flow angle is 10°. In a case where the movement distance D reaches 10 cm while the angle θ increases by 5° over a period of 5 seconds, an angle θ at the time when it increases by 5° is regarded as the gel flow rate. As a result, the gel flow angle is any one among 10° and values that are increased stepwise by 5° from 10° (for example, 15°, 20°, 25°, 30°, 35°, and 40°).

The aspect ratio of the water absorbent resin particles may be 1.0 or more and 1.5 or less. Water absorbent resin particles having an aspect ratio of 1.0 or more and 1.5 or less are particularly likely to have a gel flow angle of 10° or more and 35° or less. From the same viewpoint as above, the aspect ratio of the water absorbent resin particles may be 1.4 or less, 1.3 or less, 1.2 or less, or 1.1 or less. The water absorbent resin particles may be spherical particles having an aspect ratio within these ranges. Water absorbent resin particles having an aspect ratio of 1.5 or less can be produced, for example, by solution polymerization, suspension polymerization, or gas phase polymerization.

The aspect ratio is determined by a method including taking out, as a specimen, a fraction that passes through a JIS Z8801-1 standard sieve of 36 meshes (mesh opening: 425 μm) and is retained on a JIS Z8801-1 standard sieve of 50 meshes (mesh opening: 300 μm); imaging the specimen with a scanning electron microscope (SEM); measuring the long diameter (the maximum width of the particle in the longitudinal direction) and the short diameter (the maximum width of the particle in a direction orthogonal to the longitudinal direction) in images of fifty particles of water absorbent resin particles which are randomly selected from the obtained photographic image, and calculating the aspect ratio (= long diameter/short diameter) of each water absorbent resin particle; and calculating the average value of the aspect ratios of the fifty particles of water absorbent resin particles.

The median particle diameter of the water absorbent resin particles may be, for example, 200 μm or more and 600 μm or less, 200 μm or more and 500 μm or less, 200 μm or more and 450 μm or less, 250 μm or more and 600 μm or less, 250 μm or more and 550 μm or less, 250 μm or more and 500 μm or less, 250 μm or more and 450 μm or less, 300 μm or more and 600 μm or less, 300 μm or more and 550 μm or less, 300 μm or more and 500 μm or less, 300 μm or more and 450 μm or less, 350 μm or more and 600 μm or less, 350 μm or more and 550 μm or less, 350 μm or more and 500 μm or less, or 350 μm or more and 450 μm or less.

The median particle diameter is measured by the following method. The JIS standard sieves are assembled in the following order from top to bottom: a sieve having a mesh opening of 710 μm, a sieve having a mesh opening of 600 μm, a sieve having a mesh opening of 500 μm, a sieve having a mesh opening of 425 μm, a sieve having a mesh opening of 300 μm, a sieve having a mesh opening of 250 μm, a sieve having a mesh opening of 180 μm, and a receiving pan. 5 g of water absorbent resin particles is placed on the assembled top sieve and classified using a Continuous Automated Sonic Sieving Particle Size Analyzer (Robot Sifter RPS-205, manufactured by SEISHIN ENTERPRISE Co., Ltd.). After the classification, the mass of the particles remaining on each sieve is calculated as a mass percentage with respect to the total amount to determine the particle size distribution. With regard to this particle size distribution, the mass of particles on the sieve is integrated in descending order of particle diameter to plot, on the logarithmic probability paper, a relationship between the mesh opening of the sieve and the integrated value of the mass percentage of the particles remaining on the sieve. The plot on the probability paper is subjected to connection with a straight line to obtain, as the median particle diameter, a particle diameter that is equivalent to an integrated mass percentage of 50% by mass. Other details of the test conditions will be described in Examples described later.

The absorption against pressure (AAP) of the water absorbent resin particles at a pressure of 4.83 kPa may be 9 g/g or more, or 10 g/g or more, from the viewpoint of reducing the absolute value of the amount of re-wet. The absorption against pressure (AAP) of the water absorbent resin particles at a pressure of 4.83 kPa may be 35 g/g or less, 34 g/g or less, 33 g/g or less, 32 g/g or less, 31 g/g or less, 30 g/g or less, 29 g/g or less, 28 g/g or less, 27 g/g or less, 26 g/g or less, 25 g/g or less, 24 g/g or less, 23 g/g or less, 22 g/g or less, 21 g/g or less, 20 g/g or less, 19 g/g or less, or 18 g/g or less The absorption against pressure (AAP) of the water absorbent resin particles at a pressure of 4.83 kPa may be 9 g/g or more and 35 g/g or more. Water absorbent resin particles having an AAP within these ranges can be obtained, for example, by adjusting the amount of the surface crosslinking agent.

The water absorbent resin particles can be produced, for example, by a method which includes: forming a hydrous gel-like polymer containing an ethylenically unsaturated monomer and water, in a reaction solution containing at least one kind of ethylenically unsaturated monomer selected from the group consisting of (meth)acrylic acid and a salt thereof, a radical polymerization initiator, and water; forming polymer particles containing a polymer, from the hydrous gel-like polymer; and crosslinking the polymer in the polymer particles by a reaction with a surface crosslinking agent, in a mixture containing the polymer particles and a surface crosslinking agent.

The reaction solution for the polymerization of the ethylenically unsaturated monomer may be, for example, a homogeneous aqueous solution, or may be a dispersion liquid containing a dispersion medium (for example, a hydrocarbon compound) and liquid droplets containing water that is dispersed in the dispersion medium The dispersion liquid as the reaction solution may contain, as necessary, a surfactant and/or a polymer-based dispersant. The aqueous solution as the reaction solution or the water-containing liquid droplets in the dispersion liquid may contain a thickener such as hydroxyethyl cellulose.

The amount of the radical polymerization initiator contained in the reaction solution may be, for example, 0.30 mmol or more, 0.35 mmol or more, 0.40 mmol or more, or 0.45 mmol or more, and it may be 10 mmol or less, 5 mmol or less, 1 mmol or less, 0.8 mmol or less, 0.6 mmol or less, or 0.5 mmol or less, with respect to 1 mole of the ethylenically unsaturated monomer. The amount of the radical polymerization initiator contained in the reaction solution may be 0.30 mmol or more and 10 mmol or less with respect to 1 mole of the ethylenically unsaturated monomer. In a case where the amount of the radical polymerization initiator is within these ranges, water absorbent resin particles having a CRC and a gel flow angle within the above-described ranges are particularly easily formed.

The radical polymerization initiator contained in the reaction solution may contain an azo compound. In a case where the radical polymerization initiator contains an azo compound, water absorbent resin particles having a CRC and a gel flow angle within the above-described ranges are particularly easily formed. The amount of the azo compound in the reaction solution may be, for example, 0.25 mmol or more, 0.30 mmol or more, or 0.35 mmol or more, and it may be 5 mmol or less, 1 mmol or less, 0.8 mmol or less, 0.6 mmol or less, or 0.5 mmol or less, with respect to 1 mole of the ethylenically unsaturated monomer. The amount of the azo compound in the reaction solution may be 0.25 mmol or more and 5 mmol or less with respect to 1 mole of the ethylenically unsaturated monomer.

The radical polymerization initiator may include, for example, at least one azo compound selected from the group consisting of 2,2'-azobis(2-amidinopropane)dihydrochloride, 2,2'-azobis[2-(N-phenylamidino)propane] dihydrochloride, 2,2'-azobis[2-(N-allylamidino)propane]dihydrochloride, 2,2'-azobis[2-(2-imidazolin-2-yl)propane]dihydrochloride, 2,2'-azobis {2-[1-(2-hydroxyethyl)-2-imidazolin-2-yl]propane} dihydrochloride, 2,2'-azobis {2-methyl-N-[1,1-bis(hydroxymethyl)-2-hydroxyethyl]propane}propionamide, 2,2'-azobis[2-methyl-N-(2-hydroxyethyl)-propionamide], and 4,4'-azo-bis(4-cyanovaleric acid).

Examples of the radical polymerization initiator other than the azo compound include persulfates such as potassium persulfate, ammonium persulfate, and sodium persulfate; and peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide.

The reaction solution for the polymerization of the ethylenically unsaturated monomers may contain one or more internal crosslinking agents. In the reaction solution containing the internal crosslinking agent, a crosslinked polymer crosslinked by a reaction with the internal crosslinking agent is formed.

The internal crosslinking agent may be a compound having two or more reactive functional groups that are reactive with an ethylenically unsaturated monomer (particularly, (meth)acrylic acid and a salt thereof). The reactive functional group can be, for example, a (meth)acryloyl group, a vinyl group, an epoxy group, a halogeno group in a haloepoxy compound, an isocyanate group, or a combination thereof.

Examples of the internal crosslinking agent having two or more (meth)acryloyl groups include a (meth)acrylic acid ester compound formed from a polyol compound and (meth)acrylic acid, an unsaturated polyester formed from a polyol compound and an unsaturated carboxylic acid (maleic acid, fumaric acid, or the like), a bis(meth)acrylamide compound (N,N'-methylenebis(meth)acrylamide or the like), a (meth)acrylic acid ester compound formed from a polyepoxide compound and (meth)acrylic acid, and a (meth)acrylic acid carbamyl ester compound formed from a polyisocyanate compound (tolylene diisocyanate, hexamethylene diisocyanate, or the like) and hydroxyethyl (meth)acrylate. The polyol compound for forming the (meth)acrylic acid ester compound or the unsaturated polyester may be, for example, ethylene glycol, propylene glycol, trimethylolpropane, glycerin, polyoxyethylene glycol, polyoxypropylene glycol, polyglycerin, or a combination thereof.

The vinyl group as a reactive functional group may be a part of the allyl group. Examples of the internal crosslinking agent having two or more vinyl groups (or allyl groups) include allylated starch, allylated cellulose, diallyl phthalate, N,N',N"-triallyl isocyanurate, and divinylbenzene.

Examples of the internal crosslinking agent having two or more epoxy groups include (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether.

A haloepoxy compound has epoxy and halogeno groups as reactive functional groups, and examples thereof include epichlorohydrin, epibromohydrin, and α-methyl epichlorohydrin.

Examples of the internal crosslinking agent having two or more isocyanate groups include 2,4-tolylene diisocyanate and hexamethylene diisocyanate.

The amount of the internal crosslinking agent may be 0 mmol or more and 0.05 mmol or less, 0 mmol or more and 0.04 mmol or less, 0 mmol or more and 0.03 mmol or less, 0 mmol or more and 0.02 mmol or less, or 0 mmol or more and 0.01 mmol or less, or it may be 0 mmol, with respect to 1 mole of the ethylenically unsaturated monomer. In a case where the amount of the internal crosslinking agent is small, water absorbent resin particles having a higher CRC tend to be formed. In a case where the amount of the internal crosslinking agent is adjusted within the above-described range, water absorbent resin particles having a CRC and a gel flow angle within the above-described ranges are particularly easily formed.

A mixture that contains the hydrous gel-like polymer formed by the polymerization reaction and contains a reducing agent may be heated. By this operation, water absorbent resin particles having a gel flow angle in the above-described range may be particularly easily formed. This is conceived to be because the crosslinked polymer is partially decomposed by a reaction with the reducing agent, which results in a moderated increase in the flowability of the gel. Therefore, absorbent resin particles that form a gel having moderate flowability may also be formed by partially decomposing the crosslinked polymer according to a method other than the reaction with the reducing agent. The mixture containing the hydrous gel-like polymer and the reducing agent may have a water percentage of 150% or more. Here, the water percentage is a proportion of an amount of water to the total amount of components other than water in the mixture. The mixture containing the hydrous gel-like polymer and the reducing agent may be heated to, for example, 70°C or higher and 250°C or lower. The reducing agent may be, for example, ascorbic acid, iron (II) sulfate, or a combination thereof.

The amount of the reducing agent may be, for example, 0.001 mmol or more, 0.003 mmol or more, 0.005 mmol or more, 0.007 mmol or more, or 0.010 mmol or more, and it may be 0.500 mmol or less, 0.200 mmol or less, 0.100 mmol or less, 0.080 mmol or less, 0.060 mmol or less, 0.040 mmol or less, or 0.020 mmol or less, with respect to 1 mole of the ethylenically unsaturated monomer. The amount of the reducing agent may be 0.001 mmol or more and 0.500 mmol or less with respect to 1 mole of the ethylenically unsaturated monomer.

Polymer particles containing a polymer (or crosslinked polymer) are formed by a method that includes removing water from a hydrous gel-like polymer (or a mixture further containing a reducing agent). In a case where the reaction solution is an aqueous solution, the polymer particles are formed by a method which includes: removing water from a hydrous gel-like lumpy polymer formed by the gelation of the reaction solution itself and then forming a dried product containing a polymer (or a crosslinked polymer); and pulverizing the dried product.

Subsequently, in the mixture containing polymer particles and a surface crosslinking agent, the polymer (or crosslinked polymer) in the polymer particles may be crosslinked by a reaction with the surface crosslinking agent.

The surface crosslinking agent may be a compound having two or more reactive functional groups that are reactive with a monomer derived from an ethylenically unsaturated monomer (particularly, (meth)acrylic acid and a salt thereof). The reactive functional group of the surface crosslinking agent may be a carbonate group, an alcoholic hydroxyl group, an epoxy group, a halogeno group in a haloepoxy compound, an isocyanate group, an oxetanyl group, an oxazoline group, or a combination thereof. The carbonate group can react with two other molecules, and thus it is regarded as two reactive functional groups.

Examples of the surface crosslinking agent having a carbonate group include alkylene carbonates (ethylene carbonate and the like). Examples of the surface crosslinking agent having an alcoholic hydroxyl group include polyol compounds such as ethylene glycol, propylene glycol, 1,4-butanediol, trimethylolpropane, glycerin, polyoxyethylene glycol, polyoxypropylene glycol, and polyglycerin, as well as hydroxyalkyl amide compounds (bis[N,N-di(β-hydroxyethyl)] adipamide and the like). Examples of the surface crosslinking agent having two or more epoxy groups include (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. Examples of the haloepoxy compound having an epoxy group and a halogeno group include epichlorohydrin, epibromohydrin, and α-methyl epichlorohydrin. Examples of the surface crosslinking agent having an isocyanate group include 2,4-tolylene diisocyanate and hexamethylene diisocyanate. Examples of the surface crosslinking agent having an oxetanyl group include 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol.

The amount of the surface crosslinking agent may be, for example, 1.0 mmol or more, 1.5 mmol or more, or 2.0 mmol or more, and it may be 10 mmol or less, 8.0 mmol or less, 6.0 mmol or less, or 4.0 mmol or less, with respect to 1 mole of the ethylenically unsaturated monomer (charging amount for polymerization reaction). The amount of the surface crosslinking agent may be 1.0 mmol or more and 10 mmol or less with respect to 1 mole of the ethylenically unsaturated monomer (charging amount for polymerization reaction).

The ratio of the molar amount of the internal crosslinking agent to the molar amount of the surface crosslinking agent (molar amount of internal crosslinking agent/molar amount of surface crosslinking agent) may be 0 or more and 0.05 or less, 0 or more and 0.0026 or less, 0 or more and 0.015 or less, or 0 or more and 0.010 or less. In a case where the ratio of the molar amount of the internal crosslinking agent to the molar amount of the surface crosslinking agent is within these ranges, water absorbent resin particles having a CRC and a gel flow angle within the above-described ranges are particularly easily formed.

The polymer particles themselves which have been subjected to surface crosslinking may be used as the water absorbent resin particles. The polymer particles may contain a certain amount of water. The water absorbent resin particles may further contain an additional component introduced into the inside of the polymer particles and/or on the surface of the polymer particles. Examples of the additional component include a gel stabilizer, a metal chelating agent, and inorganic particles (silica particles or the like). The particle size distribution of the water absorbent resin particles (or polymer particles) may be adjusted as necessary. For example, a fraction of the powder of polymer particles, which passes through a sieve having a mesh opening of 850 μm, may be used as the water absorbent resin particles.

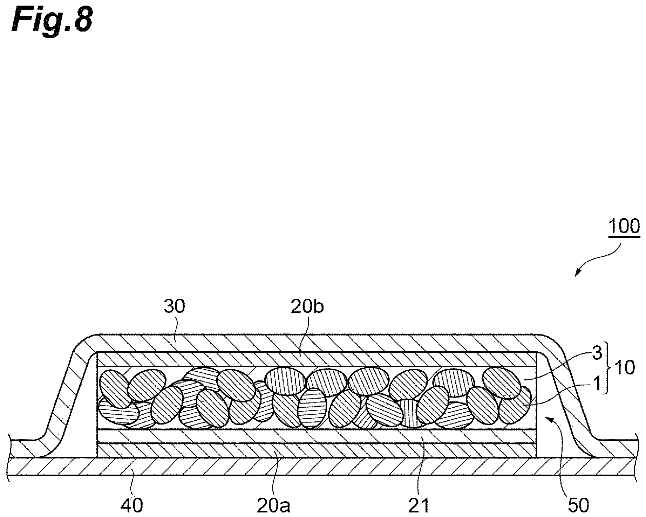

FIG. 8 is a cross-sectional view showing one example of an absorbent article having an absorber containing water absorbent resin particles. An absorbent article 100 shown in FIG. 8 includes a water absorbent sheet 50 having the film-shaped absorber 10, a liquid-permeable sheet 30, and a liquid-impermeable sheet 40.

The water absorbent sheet 50 has the absorber 10 containing a powder of water absorbent resin particles 1, and two core wrap sheets 20a and 20b. The absorber 10 is disposed inside the core wrap sheets 20a and 20b. The absorber 10 is held in terms of shape by being sandwiched between two core wrap sheets 20a and 20b. The core wrap sheets 20a and 20b may be two sheets, may be a single folded sheet, or may be a single sack body. A sheet member having no other constitutional members provided on the outer side of the core wrap sheets 20a and 20b that wrap the absorber 10 may be particularly referred to as a water absorbent sheet.

The absorber 10 is a constitutional member that mainly contains a powder of the water absorbent resin particles 1 and is held in terms of shape so that a certain shape is provided. The absorber 10 may contain a fibrous material 3 in addition to the powder of the water absorbent resin particles 1 or may not contain fibrous material 3. The content of the water absorbent resin particles 1 in the absorber 10 may be 50% by mass or more and 100% by mass or less, 60% by mass or more and 100% by mass or less, 70% by mass or more and 100% by mass or less, 80% by mass or more and 100% by mass or less, or 90% by mass or more and 100% by mass or less, based on the mass of the absorber 10.

The thickness of the absorber 10 may be, for example, 20 mm or less, 15 mm or less, 10 mm or less, 5 mm or less, 4 mm or less, or 3 mm or less, and it may be 0.1 mm or more or 0.3 mm or more. The thickness of the absorber 10 may be 0.1 mm or more and 20 mm or less. The mass per unit area of the absorber 10 may be 1,000 $g/m^2$ or less, 800 $g/m^2$ or less, or 600 $g/m^2$ or less, and it may be 100 $g/m^2$ or more.

The fibrous material 3 can be, for example, a cellulose-based fiber, a synthetic fiber, or a combination thereof. Example of the cellulose-based fiber include crushed wood pulp, cotton, a cotton linter, rayon, and cellulose acetate. Examples of the synthetic fiber include a polyamide fiber, a polyester fiber, and a polyolefin fiber. The fibrous material may be a hydrophilic fiber (for example, pulp).

The absorber 10 may further contain an inorganic powder (for example, amorphous silica), a deodorant, an antibacterial agent, a flavoring agent, and the like. In a case where the water absorbent resin particles 1 contain inorganic particles, the absorber 10 may contain an inorganic powder in addition to the inorganic particles in the water absorbent resin particles 1.

The water absorbent sheet 50 may further have an adhesive 21 that is interposed between the core wrap sheet 20a and the absorber 10. An adhesive layer may be interposed between each of the core wrap sheets 20a and 20b on both sides and the absorber 10. The adhesive 21 is not particularly limited and may be, for example, a hot melt adhesive.

The core wrap sheets 20a and 20b may be, for example, nonwoven fabric. Two core wrap sheets 20a and 20b can be the same nonwoven fabric or nonwoven fabrics different from each other. The nonwoven fabric may be a nonwoven fabric (short fiber nonwoven fabric) composed of short fibers (that is, staples) or may be a nonwoven fabric (long fiber nonwoven fabric) composed of long fibers (that is, filaments). The staple is not limited thereto; however, it may generally have a fiber length of several hundred mm or less.

The core wrap sheets 20a and 20b may be a thermal-bonded nonwoven fabric, an air-through nonwoven fabric, a resin-bonded nonwoven fabric, a spun-bonded nonwoven fabric, a melt-blown nonwoven fabric, an air-laid nonwoven fabric, a spun-lace nonwoven fabric, a point-bonded nonwoven fabric, or a laminate containing two or more kinds of nonwoven fabric selected from these.

The nonwoven fabric that is used as the core wrap sheets 20a and 20b can be a nonwoven fabric formed from synthetic fibers, natural fibers, or a combination thereof. Examples of the synthetic fiber include fibers containing a synthetic resin selected from polyolefins such as polyethylene (PE) and polypropylene (PP), polyesters such as polyethylene terephthalate (PET), polytrimethylene terephthalate (PTT), and polyethylene naphthalate (PEN), polyamides such as nylon, and rayon. Examples of the natural fiber include cotton, silk, hemp, or a fiber containing pulp (cellulose). The fiber that forms the nonwoven fabric may be a polyolefin fiber, a polyester fiber, or a combination thereof. The core wrap sheets 20a and 20b may be tissue paper.

The water absorbent sheet 50 may also be used to produce a variety of other absorbent articles. Examples of the absorbent article include a diaper (for example, a paper diaper), toilet training pants, an incontinence pad, a sanitary material (a sanitary napkin, a tampon, or the like), a sweat-absorbing pad, a pet sheet, a portable toilet member, and a treatment material for animal excrement. Absorbers constituting these absorbent articles often move or deform due to the movements of the user of the absorbent article.

The liquid-permeable sheet 30 is disposed at the outermost layer position on a side into which a liquid to be absorbed penetrates. The liquid-permeable sheet 30 is disposed on the outer side of the core wrap sheet 20b, in a state of being in contact with the core wrap sheet 20b. The liquid-impermeable sheet 40 is disposed in the absorbent article 100 at the outermost layer position on a side opposite to the liquid-permeable sheet 30. The liquid-impermeable sheet 40 is disposed on the outer side of the core wrap sheet 20a, in a state of being in contact with the core wrap sheet 20a. The liquid-permeable sheet 30 and the liquid-impermeable sheet 40 have a main surface that is wider than the main surface of the water absorbent sheet 50, and the outer edge parts of the liquid-permeable sheet 30 and the liquid-impermeable sheet 40 extend around the absorber 10 and the core wrap sheets 20a and 20b. However, a size relationship among the absorber 10, the core wrap sheets 20a and 20b, the liquid-permeable sheet 30, and the liquid-impermeable sheet 40 is not particularly limited, and it is appropriately adjusted depending on the use application of the absorbent article, and the like.

The liquid-permeable sheet 30 may be a nonwoven fabric. The nonwoven fabric that is used as the liquid-permeable sheet 30 may have moderate hydrophilicity from the viewpoint of the liquid absorption performance of the absorbent article. From this point of view, the liquid-permeable sheet 30 may be a nonwoven fabric having a hydrophilicity of 5 to 200, where the hydrophilicity is measured according to the measurement method of Paper and Pulp Testing Method No. 68 (2000) by the Japan Technical Association Of The Pulp And Paper Industry. The hydrophilicity of the nonwoven fabric may be 10 to 150. For details of Paper and Pulp Testing Method No. 68, for example, WO2011/086843 can be referred to.

The nonwoven fabric having hydrophilicity may be formed, for example, from fibers exhibiting a moderate degree of hydrophilicity, such as rayon fibers, or it may be formed from fibers obtained by subjecting hydrophobic chemical fibers, such as polyolefin fibers and polyester fibers, to a hydrophilization treatment. Examples of the method for obtaining a nonwoven fabric containing hydrophobic chemical fibers that have been subjected to a hydrophilization treatment include a method in which hydrophobic chemical fibers mixed with a hydrophilizing agent are used to obtain a nonwoven fabric according to a spun bonding method, a method in which a hydrophilizing agent is accompanied in a case where a spun-bonded nonwoven fabric is prepared with hydrophobic chemical fibers, and a method in which a spun-bonded nonwoven fabric obtained using hydrophobic chemical fibers is impregnated with a hydrophilizing agent. As the hydrophilizing agent, the following hydrophilizing agents are used: anionic surfactants such as an aliphatic sulfonic acid salt and a higher alcohol sulfuric acid ester salt, cationic surfactants such as a quaternary ammonium salt; nonionic surfactants such as a polyethylene glycol fatty acid ester, a polyglycerin fatty acid ester, and a sorbitan fatty acid ester, silicone-based surfactants such as polyoxyalkylene-modified silicone, stain release agents consisting of a resin which is polyester-based, polyamide-based, acrylic, or urethane-based, and the like.

The areal weight (mass per unit area) of the nonwoven fabric that is used as the liquid-permeable sheet 30 may be 5 to 200 g/m$^2$, 8 to 150 g/m$^2$, or 10 to 100 g/m$^2$ from the viewpoint of imparting favorable liquid permeability, flexibility, strength, and cushioning properties to the absorbent article and from the viewpoint of increasing the liquid permeation rate of the absorbent article. The thickness of the liquid-permeable sheet 30 may be 20 to 1400 μm, 50 to 1200 μm, or 80 to 1000 μm.

The liquid-impermeable sheet 40 prevents a liquid absorbed in the absorber 10 from leaking out from the liquid-impermeable sheet 40 side to the outside. The liquid-impermeable sheet 40 may be a resin sheet or a nonwoven fabric. The resin sheet may be a sheet consisting of a synthetic resin such as polyethylene, polypropylene, or polyvinyl chloride. The nonwoven fabric may be a spun-bonded/melt-blown/spun-bonded (SMS) nonwoven fabric, in which a water-resistant melt-blown nonwoven fabric is sandwiched between high-strength spun-bonded nonwoven fabrics. The liquid-impermeable sheet 40 may be a composite sheet of a resin sheet and a nonwoven fabric (for example, a spun-bonded nonwoven fabric or a spun-lace nonwoven fabric). From the viewpoint of, for example, reducing stuffiness during mounting, thereby being capable of reducing a feeling of discomfort of a user, the liquid-impermeable sheet 40 may have air permeability. As the liquid-impermeable sheet 40 having air permeability, it is possible to use, for example, a sheet of low density polyethylene (LDPE) resin.

From the viewpoint of ensuring flexibility so that the wear feeling of the absorbent article is not impaired, the areal weight (mass per unit area) of the liquid-impermeable sheet 40 may be 10 to 50 g/m$^2$.

EXAMPLES

The present invention is not limited to Examples below.

1. Water absorbent resin particles

Example 1

A round-bottom cylindrical separable flask having an inner diameter of 11 cm and an internal volume of 2 L, which was equipped with a reflux condenser, a dropping funnel, a nitrogen gas introduction pipe, and a stirrer, was prepared. The stirrer had a stirring blade that had, in two stages, four inclined paddle blades having a blade diameter of 5 cm. 300 g of n-heptane, 0.736 g of a maleic anhydride-modified ethylene-propylene copolymer (Hi-WAX 1105A, Mitsui Chemicals, Inc.) as a polymer-based dispersant, and 0.736 g of a sucrose stearic acid ester (HLB: 3, RYOTO Sugar Ester S-370, Mitsubishi-Chemical Foods Corporation) as a surfactant were placed in the separable flask. The mixture in the separable flask was heated to 80°C while being stirred at a rotation speed of 400 rpm, and then a dispersant and a surfactant were dissolved in n-heptane. The formed dispersion medium was cooled to 60°C.

92.0 g (1.03 mol) of an aqueous solution of acrylic acid having a concentration of 80.5% by mass was placed in a beaker having an internal volume of 300 mL. While carrying out cooling from the outside, 103.63 g of an aqueous solution of sodium hydroxide having a concentration of 30% by mass was added dropwise to the aqueous solution of acrylic acid, thereby adjusting a partially neutralized solution of acrylic acid, in which 75% by mole of acrylic acid was neutralized. To the aqueous solution after the adjustment, 1.38 g of hydroxyethyl cellulose (Sumitomo Seika Chemicals Co., Ltd., HECAW-15F) as a thickener, 0.1105 g (0.407 mmol) of 2,2'-azobis(2-amidinopropane)dihydrochloride as an azo-based compound, and 42.83 g of ion exchange water were added to prepare an aqueous monomer solution.

The aqueous monomer solution was added to the dispersion medium in the separable flask, and the atmosphere in the system was thoroughly replaced with nitrogen while stirring the reaction solution containing the dispersion medium and the aqueous monomer solution. Thereafter, the separable flask was immersed in a water bath at 70°C, and a polymerization reaction was allowed to proceed for 30 minutes.

While stirring, at a rotation speed of 1,000 rpm, a reaction solution (polymerization slurry liquid) containing the hydrous gel-like polymer formed by the polymerization reaction, 0.18 g (0.051 mmol) of an aqueous solution containing ascorbic acid at a concentration of 5% by mass as a reducing agent was sprayed into the reaction solution using a 3 mL syringe equipped Fine Atomizer (oral, manufactured by Yoshikawa Kasei Co., Ltd.). The water percentage of the hydrous gel-like polymer before the aqueous solution of ascorbic acid was sprayed was 159% by mass. The water percentage was calculated according to the following expression.

$$\text{water percentage [\% by mass]} = (\text{Ww/Ws}) \times 100$$

Ww: water amount in aqueous monomer solution before mixing with dispersion medium
Ws: Total amount of charging amounts of ethylenically unsaturated monomer, thickener, crosslinking agent, and radical polymerization initiator

After the aqueous solution of ascorbic acid was sprayed, the separable flask was immersed in an oil bath set at 125°C. While refluxing n-heptane by azeotropic distillation of n-heptane and water, 117.59 g of water was extracted to the outside of the system. Next, 5.52 g (3.166 mmol) of an aqueous solution of ethylene glycol diglycidyl ether having a concentration of 10% by mass as a surface crosslinking agent was placed in the separable flask, and the internal temperature of the separable flask was held at 80°C for 2 hours.

Thereafter, the separable flask was immersed in an oil bath set at 125°C. The n-heptane was vaporized to obtain a dried product of polymer particles remaining in the separable flask. The polymer particles were allowed to pass through a sieve having a mesh opening of 850 μm. 92.8 g of polymer particles that passed through the sieve were obtained as water absorbent resin particles of Example 1.

Example 2

85.0 g of water absorbent resin particles of Example 2 was obtained in the same manner as in Example 1, except that 0.0028 g (0.016 mmol) of ethylene glycol diglycidyl ether was added to the aqueous monomer solution as an internal crosslinking agent, that the amount of ion exchange water in the aqueous monomer solution was changed to 43.11 g, that the aqueous solution of the reducing agent (ascorbic acid) was changed to 0.04 g (0.013 mmol) of an aqueous solution of iron (II) sulfate having a concentration of 5% by mass, that the amount of water extracted to the outside of the system by azeotropic distillation was changed to 117.07 g, and that the amount of the surface crosslinking agent (an aqueous solution of ethylene glycol diglycidyl ether) was changed to 3.68 g (2.110 mmol).

Example 3

92.0 g of water absorbent resin particles of Example 3 was obtained in the same manner as in Example 1, except that the aqueous solution of the reducing agent was changed to 0.04 g (0.013 mmol) of an aqueous solution of 5% by mass iron (II) sulfate, and that the amount of water extracted to the outside of the system by azeotropic distillation was changed to 120.17 g.

Comparative Example 1

A round-bottom cylindrical separable flask having an inner diameter of 11 cm and an internal volume of 2 L, which was equipped with a reflux condenser, a dropping funnel, a nitrogen gas introduction pipe, and a stirrer, was prepared. The stirrer had four two-staged and inclined paddle blades having a blade diameter of 5 cm. 293 g of n-heptane and 0.736 g of a maleic anhydride-modified ethylene-propylene copolymer (Hi-WAX 1105A, manufactured by Mitsui Chemicals, Inc.) as a polymer-based dispersant were placed in the separable flask. The mixture in the separable flask was heated to 80°C while being stirred at a rotation speed of 300 rpm, and then a dispersant was dissolved in n-heptane. The formed dispersion medium was cooled to 50°C.

92.0 g (1.03 mol) of an aqueous solution of acrylic acid having a concentration of 80.5% by mass was placed in a beaker having an internal volume of 300 mL. While carrying out cooling from the outside, 103.63 g of an aqueous solution of sodium hydroxide having a concentration of 30% by mass was added dropwise to the aqueous solution of acrylic acid, thereby adjusting a partially neutralized solution of acrylic acid, in which 75% by mole of acrylic acid was neutralized. 0.092 g of hydroxyethyl cellulose (Sumitomo Seika Chemicals Co., Ltd., HECAW-15F) as a thickener, 0.0736 g (0.272 mmol) of potassium persulfate as a radical polymerization initiator, and 0.0101 g (0.058 mmol) of ethylene glycol diglycidyl ether as an internal crosslinking agent were added to the aqueous solution after the adjustment, and these were dissolved to prepare a first-stage aqueous monomer solution.

The first-stage aqueous monomer solution was added to the dispersant in the separable flask, and the reaction solution containing the dispersion medium and the aqueous monomer solution was stirred for 10 minutes. Next, 6.62 g of n-heptane and a surfactant solution containing 0.736 g of a sucrose stearic acid ester (RYOTO Sugar Ester S-370, HLB: 3, Mitsubishi-Chemical Foods Corporation) as a surfactant were added to the reaction solution. While stirring the reaction solution at a rotation speed of 500 rpm, the atmosphere in the system was thoroughly replaced with nitrogen. The separable flask was immersed in a water bath at 70°C, and a polymerization reaction was allowed to proceed for 60 minutes to obtain a first-stage polymerization slurry liquid.

As a solution distinct from the first-stage aqueous monomer solution, 128.8 g (1.44 mol) of an aqueous solution of acrylic acid having a concentration of 80.5% by mass was placed in a beaker having an internal volume of 500 mL. While carrying out cooling from the outside, 145.1 g of an aqueous solution of sodium hydroxide having a concentration of 30% by mass was added dropwise to the aqueous solution of acrylic acid, thereby adjusting a partially neutralized solution of acrylic acid, in which 75% by mole of acrylic acid was neutralized. To the aqueous solution after the adjustment, 0.1030 g (0.381 mmol) of an adding amount of potassium persulfate, 0.0116 g (0.067 mmol) of ethylene glycol diglycidyl ether as an internal crosslinking agent, and 16.02 g of ion exchange water were added to prepare a second-stage aqueous monomer solution in which ethylene glycol diglycidyl ether was dissolved.

The first-stage polymerization slurry liquid in the separable flask was cooled to 23°C while being stirred at a rotation speed of 1,000 rpm. The entire amount of the second-stage aqueous monomer solution was added thereto. After the atmosphere inside the separable flask was replaced with nitrogen for 30 minutes, the separable flask was immersed in a water bath at 70°C, and a second-stage polymerization reaction was allowed to proceed for 60 minutes to form a hydrous gel-like polymer.

The separable flask containing the hydrous gel-like polymer was immersed in an oil bath set at 125°C, and 272.5 g of water was extracted to the outside of the system by the azeotropic distillation of n-heptane and water. Next, 4.42 g (0.507 mmol) of an aqueous solution of ethylene glycol diglycidyl ether having a concentration of 2% by mass as a surface crosslinking agent was placed in the separable flask, and the internal temperature of the separable flask was held at 83°C for 2 hours.

Thereafter, the separable flask was immersed in an oil bath set at 125°C. The n-heptane was vaporized to obtain polymer particles (dry product). The polymer particles were allowed to pass through a sieve having a mesh opening of 850 μm to obtain 231.5 g of polymer particles that passed through the sieve were obtained as water absorbent resin particles of Comparative Example 1.

2. Evaluation

Median particle diameter

The median particle diameter of the water absorbent resin particles was measured in an environment of room temperature (25°C ± 2°C) and a relative humidity of 50% ± 10% according to the following procedure. Using a Continuous Automated Sonic Sieving Particle Size Analyzer (Robot Sifter RPS-205, manufactured by SEISHIN ENTERPRISE Co., Ltd.), the particle size distribution of 5 g of water absorbent resin particles was measured using sieves of 710 μm, 600 μm, 500 μm, 425 μm, 300 μm, 250 μm, and 180 μm according to the JIS standard, and a receiving pan. With regard to this particle size distribution, the mass of particles on the sieve was integrated in descending order of particle diameter to plot, on the logarithmic probability paper, a relationship between the mesh opening of the sieve and the integrated value of the mass percentage of the particles remaining on the sieve. The plot on the probability paper was subjected to connection with a straight line to obtain, as the median particle diameter, a particle diameter that is equivalent to an integrated mass percentage of 50% by mass.

Aspect ratio

From the powder of water absorbent resin particles, a fraction that passed through a standard sieve of 36 meshes (mesh opening: 425 μm) and was retained on a JIS Z8801-1 standard sieve of 50 meshes (mesh opening: 300 μm) was taken out as a specimen. This specimen was imaged with a scanning electron microscope (SEM). The long diameter (the maximum width of the particle in the longitudinal direction) and the short diameter (the maximum width of the particle in a direction orthogonal to the longitudinal direction) of 50 water absorbent resin particles which were randomly selected from the obtained photographic image were measured. The aspect ratio of each water absorbent resin particle was calculated from the long diameter and short diameter thereof. The average value of the obtained aspect ratios of the 50 particles was defined as the aspect ratio (= long diameter/short diameter) of the water absorbent resin particle.

Centrifuge retention capacity (CRC)

The centrifuge retention capacity (CRC) was measured according to the following procedure with reference to the EDANA method (NWSP 241.0.R2 (15), pages 769-778). The measurements were carried out in an environment of a temperature of 25°C ± 2°C and a relative humidity of 50% ± 10%.
A nonwoven fabric having a size of 60 mm × 170 mm (product name: Heat Pack MWA-18, manufactured by NIPPON PAPER PAPYLIA CO., LTD.) was folded in half in the longitudinal direction. The nonwoven fabrics were subjected to pressure bonding to each other by heat sealing at the respective 5 mm-wide end parts along the two long sides, thereby preparing a nonwoven fabric bag having a size of 60 mm × 85 mm. Approximately 0.2 g of precisely weighed particles to be measured was accommodated in the nonwoven fabric bag. The nonwoven fabrics were subjected to pressure bonding to each other by heat sealing at the opened end part on the side of the short side, thereby closing the nonwoven fabric bag.
A plurality of nonwoven fabric bags accommodating the particles were floated on 1,000 g of saline accommodated in a stainless steel vat (240 mm × 320 mm × 45 mm) so that the plurality of nonwoven fabric bags did not fold with each other, thereby completely wetting the entire nonwoven fabric bag. 1 minute after the nonwoven fabric bag was floated in the saline, the entire nonwoven fabric bag was immersed in the saline using a spatula.
After elapsing 30 minutes after the nonwoven fabric bag started to be floated in the saline, that is, after elapsing a total time of the time of 1 minute for floating the nonwoven bag and 29 minutes of the immersion time, the nonwoven fabric bag containing the formed gel was taken out from the saline. The taken-out nonwoven fabric bag was dehydrated for 3 minutes at a centrifugal force of 250 G with a centrifuge (manufactured by KOKUSAN Co. Ltd., model number: H-122). After the dehydration, a mass Ma [g] of the nonwoven fabric bag, including the mass of the gel, was weighed. A nonwoven fabric bag accommodating no particles to be measured was subjected to the above-described operation to measure a mass Mb [g] of the nonwoven fabric bag after the dehydration. The CRC [g/g] was calculated according to the following expression. Mc [g] is a precisely weighed value of 0.2 g of the mass of the particles to be measured.

$$CRC \ [g/g] = \{(Ma - Mb) - Mc\}/Mc$$

Absorption against pressure (AAP)

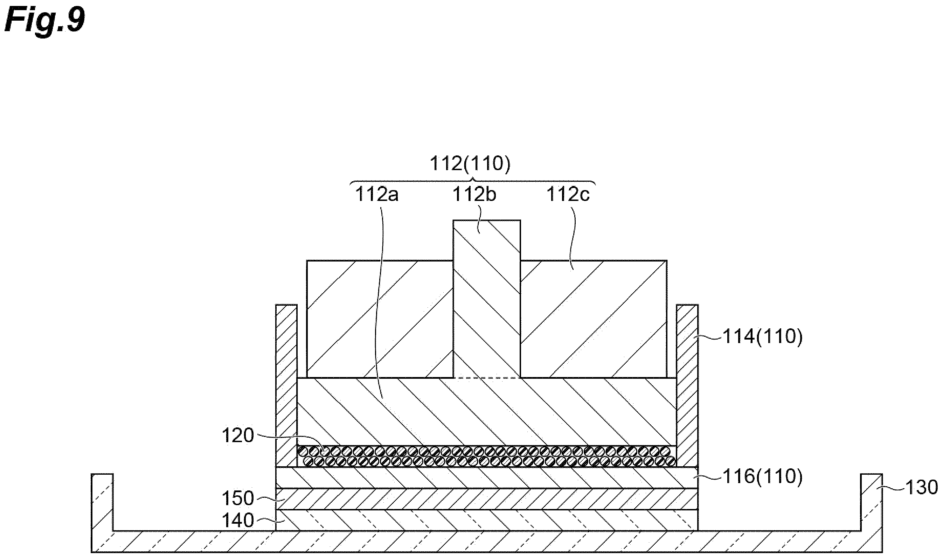

Using a measuring device 110 shown in FIG. 9, the absorption against pressure (AAP) at a pressure of 4.83 kPa was measured in an environment of a temperature of 25°C ± 2°C and a relative humidity of 50% ± 10%. The measuring device 110 is composed of a weight 112, a plastic cylinder 114 having an inner diameter of 60 mm, and a wire net 116 of 400 meshes (mesh opening: 38 μm). The cylinder 114 and the wire net 116 are disposed so that the wire net 116 blocks one opening of the cylinder 114, and the wire net 116 is horizontal. The weight 112 has a disk part 112a, a rod-shaped part 112b extending from the center of the disk part 112a in a direction perpendicular to the disk part 112a, and a columnar part 112c having a through hole in the center. The rod-shaped part 112b is inserted into the through hole of the columnar part 112c. The disk part 112a has a diameter substantially equal to the inner diameter of the cylinder 114 so that the disk part 112a is movable in the longitudinal direction of the cylinder 114, in the inside of the cylinder 114. The diameter of the columnar part 112c is smaller than the diameter of the disk part 112a. The weight of the weight 112 is adjusted so that a pressure of 4.83 kPa is applied to the particles to be measured. In the inside the cylinder 114, 0.90 g of particles 120 (water absorbent resin particles) to be measured was uniformly spread on the wire net 116. The weight 112 inserted into the cylinder 114 was placed on the spread particles. In this state, a total mass Wa [g] of the measuring device 110 and the particles 120 before liquid absorption was measured.

A disk-shaped glass filter 140 (ISO 4793P-250) having a diameter of 90 mm and a thickness of 7 mm was placed in the center of a bottom surface (diameter: 150 mm) of a recessed part of a stainless steel petri dish 130. Next, an aqueous solution of 0.90% by mass of sodium chloride (25°C ± 2°C) was placed in the stainless steel petri dish 130 until the water surface reached the same height as the upper surface of the glass filter 140. One sheet of Filter Paper 150 having a diameter of 90 mm (ADVANTEC TOYO KAISHA, LTD., product name: (No. 3), thickness: 0.23 mm, retaining particle diameter: 5 μm) was placed on the glass filter 140, and the entire surface of the filter paper 150 was wetted with an aqueous solution of chloride solution to remove the excessive sodium chloride solution. Subsequently, the measuring device 110 charged with the particles 120 before liquid absorption was placed on the filter paper 150, and the particles 120 were allowed to absorb the aqueous solution of sodium chloride while pressurizing the particles 120 at a pressure of 4.83 kPa. One hour after the start of absorption, the measuring device 110 was lifted up, and a total mass Wb [g] of the measuring device 110 and the particles 120 after liquid absorption was measured. From Wa and Wb, the absorption against pressure (AAP) [g/g] was calculated according to the following expression.

$$AAP\ [g/g] = (Wb - Wa)/0.90$$

Gel flow angle

According to the method shown in FIGS. 1 to 6, the gel flow angle was measured in an environment of a temperature of 25°C ± 2°C and a relative humidity of 50% ± 10%. The acrylic plate 31 (thickness: 3 mm) having, as the working surface W, a rectangular main surface having the short side 31S of 20 cm and the long side 31L of 40 cm was placed on a horizontal flat surface H of a work bench. The acrylic resin guide frame 32 (having a rectangle shape of 16 cm × 12, thickness: 2 cm) made of an acrylic resin, which has the rectangular opening 32A having the long side 32L of 12 cm and the short side 32S of 8 cm, was placed on the work surface W of the acrylic plate 31, in a direction in which the short side 31S of the acrylic plate 31 and the short side 32S of the opening 32A of the guide frame 32 were parallel to each other. The distance from one short side 31S of the working surface W of the acrylic plate 31 to the guide frame 32 was 10.5 cm. 3.00 ± 0.01 g of a powder of the water absorbent resin particles was uniformly spread over the entire inside of the opening 32A of the guide frame 32 to form the film-shaped absorber 10 consisting of water absorbent resin particles.

40 mL of saline was charged at a rate of 8 mL/s toward the central part 10C of the absorber 10 in the opening 32A of the guide frame 32, and 5 minutes thereafter, 40 mL of saline was charged again at a rate of 8 mL/s. The saline was charged using a burette. 5 minutes after the second charge of saline, the weight 33 of 222 g made of an acrylic resin, which had a rectangular main surface having a long side of 11.9 cm and a short side of 7.9 cm, was placed on the gel film 11 formed in the opening 32A of the guide frame 32. 1 minute after the weight 33 was placed, the weight 33 and the guide frame 32 were slowly removed so that the gel film 11 did not move. The wall 34 (length: 15 cm, thickness: 2 cm, height: 3 cm) made of an acrylic resin, which was parallel to the long side of the acrylic plate 31, was attached at a position 1 cm away from the two long sides 11L of the gel film 11. The end part 31SE of one short side 31S of the acrylic plate 31 was lifted up at a constant speed over a period of 5 seconds in a state where an end part on a side opposite to the end part 31SE was in contact with the flat surface H of the work bench, until the angle θ between the acrylic plate 31 and the horizontal plane (flat surface H) reached 10°, and the acrylic plate 31 was fixed in that position. 12 minutes after the first charge of saline, the weight 33 was gently placed on the upper side of the gel film 11 so that the entire end surface of the weight 33 on the side of the short side came into contact with the end surface 11SF of the gel film 11 on the side of the short side. Thereafter, every 2 minutes, the angle θ was increased by 5° over a period of 5 seconds. An angle θ at the time when the weight 33 had descended by 10 cm on the working surface W while pushing the gel film 11 was recorded as the gel flow angle [°].

Amount of re-wet of absorbent article

(1) Preparation of absorbent article

Two sheets of tissue paper having a size of 12 cm × 40 cm and having an areal weight of 16 g/m² were prepared as core wrap sheets. 0.3 g of an adhesive (3M Spray Adhesive 77, manufactured by 3M Japan Limited) was evenly applied onto the entire surface of one core wrap sheet. 10.0 g of water absorbent resin particles were quickly and uniformly spread thereon. 0.3 g of an adhesive (3M Spray Adhesive 77, manufactured by 3M Japan Limited) was evenly applied onto the entire surface of the other core wrap sheet. The second core wrap sheet coated with the adhesive was overlaid on the water absorbent resin particles on the first core wrap sheet in a direction in which the surface coated with the adhesive was in contact with the water absorbent resin particles. The absorbent sheet composed of two core wrap sheets and an absorber which is a powder of water absorbent resin particles disposed in a film shape was cut at a position 15 cm from the central part in the longitudinal direction to obtain a water absorbent sheet having a length of 30 cm. This water absorbent sheet was sandwiched between two air-through type porous liquid-permeable sheets made of polyethylene, which had an areal weight of 22 g/m² and a size of 33 cm × 15 cm, and the four sides of the two liquid-permeable sheets sandwiching the absorber were subjected to pressure bonding with a heat sealer (FUJI IMPULSE SEALER, model number: FI-450-5, manufactured by FUJI IMPULSE CO., LTD.). On one of the liquid permeable sheets, an air-through type porous liquid-permeable sheet made of polyethylene-polypropylene, which had a size of 12 cm × 30 cm and a basis weight of 22 g/m² was disposed as a top sheet to obtain an absorbent article for evaluation.

(2) Preparation of artificial urine

An artificial urine containing the following components was prepared.

· Deionized water: 5,919.6 g
· NaCl: 60.0 g
. $CaCl_2$ $H_2O$: 1.8 g
· $MgCl_2$ $6H_2O$: 3.6 g
· Food Blue No. 1 (for coloring)
· 1% Triton X-100: 15.0 g

(3) Amount of re-wet

The amount of re-wet of the absorbent article at the initial stage and after the application of centrifugal force was measured according to the following procedure. The measurement was carried out in a room regulated at 25°C ± 2°C and 50% ± 10% of relative humidity.

(Initial stage)

The absorbent article for evaluation was placed on a horizontal plane of a work bench in a direction in which the top sheet was located on the upper side. A liquid-charge cylinder having a volume of 200 mL and having an inlet with an inner diameter of 3 cm was placed toward the central part of the absorbent article, and 200 mL of the artificial urine was subjected to the first charge into a cylinder at one time. 30 minutes after the start of the first charge of the artificial urine charge, 50 mL of the artificial urine was subjected to the second charge using a cylinder toward the central part of the absorbent article. 10 minutes after the start of the second charge of the artificial urine charge, 40 sheets of square filter paper, which were 10 cm square, were placed in the central part of the absorbent article (the position where the artificial urine was charged), and a weight having a mass of 5 kg and having a bottom surface of 10 cm × 10 cm was placed thereon. 5 minutes after the weight was placed, the weight was removed, and a total mass We [g] of the 40 sheets of filter paper that had absorbed the artificial urine in the absorbent article was measured. From the We and the total mass Wd [g] of the 40 sheets of filter paper before being placed on the absorbent article, the amount of re-wet was determined according to the following expression.

$$\text{Amount of re-wet[g]} = \text{We} - \text{Wd}$$

(After application of centrifugal force)

The absorbent article for evaluation was placed on a horizontal plane of a work bench in a direction in which the top sheet was located on the upper side. A liquid-charge cylinder having a volume of 200 mL and having an inlet with an inner diameter of 3 cm was placed in the central part of the absorbent article, and 200 mL of the artificial urine was subjected to the first charge into a cylinder at one time.

13 minutes after the start of the first charge of the artificial urine charge, the absorbent article was placed on a piece of cardboard having the same size as the absorbent article (areal weight: 3,500 $g/m^2$) and an adhesive tape was attached to both end parts of the absorbent article in the longitudinal direction to fix the absorbent article to the cardboard. The absorbent article fixed to the cardboard was placed in a polyethylene sack attached with a zipper (40 cm×28 cm, thickness: 0.04 mm, UNIPACK, manufactured by SEISANNIPPONSHA LTD., K-4). The sack had three slits of 1 cm for releasing air, which had been formed in advance at three positions. The absorbent article was placed in the sack so that one corner of the article was stuffed into the corner part of the sack. A portion of the sack, which protruded from the absorbent article, was folded back at the position of the end part of the absorbent article, and in this state, the sack was fixed with an adhesive tape.

The absorbent article accommodated in the sack was fixed to a turntable of a centrifuge with an adhesive tape. The absorbent article was fixed to the turntable so that the main surface thereof was perpendicular to the rotation axis of the centrifuge and the center thereof was located on the rotation axis of the centrifuge. For 5 minutes from the elapse of 15 minutes after the start of the first charge of the artificial urine charge, a centrifugal force of 6.7 G (rotation speed of centrifuge: 200 rpm) was applied to the absorbent article for evaluation in order to move or deform the absorber.

Thereafter, the absorbent article was taken out from the sack and placed on a horizontal plane of a work bench. 30 minutes after the start of the first charge of the artificial urine charge, 50 mL of the second artificial urine was subjected to the second charge of the artificial urine using a liquid-charge cylinder, at the same position of the absorbent article as the position where the first charge of the artificial urine carried out. Thereafter, the amount of re-wet after the application of centrifugal force was measured according to the same procedure as in the measurement of the initial amount of re-wet.

Table 1

| | Water absorbent resin particle | | | | | Amount of re wet(g) | | |
|---|---|---|---|---|---|---|---|---|
| | Median particle diameter (μm) | Aspect ratio | CRC [g/g] | AAP [g/g] | Gel flow angle [°] | Initial stage $X_0$ | After application of centrifugal force $X_1$ | Degree of deterioration $X_1 - X_0$ |
| Ex. 1 | 401 | 1.0 | 45 | 18 | 20 | 5.9 | 8.0 | 2.1 |
| Ex. 2 | 377 | 1.0 | 49 | 10 | 25 | 5.2 | 5.4 | 0.2 |
| Ex. 3 | 384 | 1.0 | 35 | 28 | 35 | 23.5 | 23.6 | 0.1 |
| Comp. Ex. 1 | 349 | 1.8 | 44 | 8 | 40 | 18.9 | 31.5 | 12.6 |

The evaluation results are shown in Table 1. It has been confirmed that the water absorbent resin particles of each of Examples, which have a CRC of 30 g/g or more and a gel flow angle of 10° or more and 35° or less can suppress an increase in the amount of re-wet(degree of deterioration: $X_1 - X_0$) due to the application of centrifugal force.

**Reference Signs List**

1: Water absorbent resin particles; 10: Absorber; 11: Gel film; 11SF: End surface on side of short side of gel film 11; 31: Acrylic plate; 31L: Long side of working surface W; 31S: Short side of working surface W; 31SE: End part on side of short side 31S of acrylic plate 31; 32: Guide frame; 32A: Opening; 32L: Long side of opening 32A; 32S: Short side of opening 32A; 33, 112: Weight; 50: Water absorbent sheet; 100: Absorbent article; W: Working surface

**Claims**

1. Water absorbent resin particles comprising:

a crosslinked polymer comprising, as a monomer unit, at least one kind of ethylenically unsaturated monomer selected from the group consisting of (meth)acrylic acid and salts thereof,

wherein the water absorbent resin particles have a centrifuge retention capacity of 30 g/g or more and a gel flow angle of 10° or more and 35° or less, and

the gel flow angle is measured in an environment of a temperature of 25°C $\pm$ 2°C and a relative humidity of 50% $\pm$ 10%, according to a method which includes;

disposing an acrylic plate that has a rectangular working surface having a long side and a short side such that the working surface is horizontal,

placing a guide frame that has a thickness of 2 cm and has a rectangular opening having a long side of 12 cm and a short side of 8 cm on the working surface such that the short side of the opening is parallel to the short side of the working surface,

forming, in the opening, a film-shaped absorber consisting of 3.00 $\pm$ 0.01 g of a powder of the water absorbent resin particles,

subjecting the absorber in the opening to first charge with 40 mL of saline at a speed of 8 m/sec, and 5 minutes after start of the first charge of the saline, subjecting the absorber to second charge with 40 mL of saline at a speed of 8 m/sec, thereby forming, on the working surface in the opening, a gel film having a rectangular main surface having a long side of 12 cm and a short side of 8 cm parallel to the short side of the working surface,

5 minutes after start of the second charge of the saline, placing, on the gel film, a plate-shaped weight that weighs 222 g and has a rectangular main surface having a long side of 11.9 cm and a short side of 7.9 cm,

1 minute after the weight is placed on the gel film, removing the weight and the guide frame,

raising an end part of the acrylic plate on one side of the short side of the working surface, thereby tilting the acrylic plate and the gel film over a period of 5 seconds until an angle θ between the working surface and a horizontal plane is 10°,

12 minutes after the start of the first charge of the saline, placing the weight on the working surface on an upper side of the tilted gel film such that an entire end surface of the weight on a side of the short side comes into contact with an end surface of the gel film on a side of the short side,

every 2 minutes after the weight is placed on the working surface, further raising the end part of the acrylic plate such that the angle θ increases by 5° over a period of 5 seconds, and

recording, as a gel flow angle, an angle θ at a time when the weight has moved on the working surface 10 cm downward along a longitudinal direction of the working surface while pushing the gel film.

2. The water absorbent resin particles according to Claim 1, wherein the water absorbent resin particles have a median particle diameter of 200 μm or more and 600 μm or less.

3. The water absorbent resin particles according to Claim 1, wherein the water absorbent resin particles have an aspect ratio of 1.0 or more and 1.5 or less.

4. The water absorbent resin particles according to Claim 2, wherein the water absorbent resin particles have an aspect ratio of 1.0 or more and 1.5 or less.

5. An absorber comprising:
the water absorbent resin particles according to any one of Claims 1 to 4.

6. The absorber according to Claim 5, wherein a content of the water absorbent resin particles is 90% by mass or more and 100% by mass or less based on a mass of the absorber.

7. An absorbent article comprising:
the absorber according to Claim 5.

# *Fig.1*

(a1)

(a2)

# Fig.2

(b)

(c)

# Fig.3

(d1)

11L  34  11S

d2

d2

31S

31(W)  11  34

(d2)

31  W  11  11SF

H

# Fig.4

(e)

# Fig.5

(f1)

(f2)

# Fig.6

(g1)

(g2)

# Fig.7

# Fig.8

Fig.9

*Fig.9*

<div style="text-align: center">**INTERNATIONAL SEARCH REPORT**</div>

| | International application No. |
|---|---|
| | **PCT/JP2023/010167** |

**A. CLASSIFICATION OF SUBJECT MATTER**

*C08J 3/14*(2006.01)i; *A61F 13/53*(2006.01)i; *C08F 2/32*(2006.01)i; *C08F 8/50*(2006.01)i; *C08F 220/06*(2006.01)i
FI: C08J3/14 CEY; A61F13/53 300; C08F2/32; C08F8/50; C08F220/06

According to International Patent Classification (IPC) or to both national classification and IPC

**B. FIELDS SEARCHED**

Minimum documentation searched (classification system followed by classification symbols)

C08J3/14; A61F13/53; C08F2/32; C08F8/50; C08F220/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2020/129594 A1 (SUMITOMO SEIKA CHEMICALS CO., LTD.) 25 June 2020 (2020-06-25)<br>claims, paragraphs [0006], [0038], [0047], [0087], [0088], [0103]-[0160], examples | 1-7 |
| A | WO 2012/033025 A1 (SUMITOMO SEIKA CHEMICALS CO., LTD.) 15 March 2012 (2012-03-15)<br>claims, examples, entire text | 1-7 |
| A | JP 2012-144608 A (SUMITOMO SEIKA CHEMICALS CO., LTD.) 02 August 2012 (2012-08-02)<br>claims, examples, entire text | 1-7 |
| A | WO 2020/122210 A1 (SUMITOMO SEIKA CHEMICALS CO., LTD.) 18 June 2020 (2020-06-18)<br>claims, examples, entire text | 1-7 |
| A | WO 2020/184389 A1 (SUMITOMO SEIKA CHEMICALS CO., LTD.) 17 September 2020 (2020-09-17)<br>claims, examples, entire text | 1-7 |

☐ Further documents are listed in the continuation of Box C.　　　☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **25 May 2023** | **06 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

| International application No. |
| --- |
| **PCT/JP2023/010167** |

| Patent document cited in search report | | | Publication date (day/month/year) | Patent family member(s) | | | Publication date (day/month/year) |
| --- | --- | --- | --- | --- | --- | --- | --- |
| WO | 2020/129594 | A1 | 25 June 2020 | US | 2022/0008894 | A1 | |
| | | | | claims, paragraphs [0006], [0043], [0053], [0094], [0095], [0113]-[0182], examples | | | |
| | | | | EP | 3901182 | A1 | |
| | | | | CN | 113195548 | A | |
| | | | | KR | 10-2021-0100141 | A | |
| WO | 2012/033025 | A1 | 15 March 2012 | JP | 2016-55193 | A | |
| | | | | US | 2013/0158495 | A1 | |
| | | | | claims, examples, entire text | | | |
| | | | | JP | 2018-59124 | A | |
| | | | | EP | 2615117 | A1 | |
| | | | | CN | 103080139 | A | |
| | | | | KR | 10-2013-0140660 | A | |
| | | | | TW | 201221525 | A | |
| JP | 2012-144608 | A | 02 August 2012 | (Family: none) | | | |
| WO | 2020/122210 | A1 | 18 June 2020 | JP | 2020-93066 | A | |
| | | | | US | 2022/0015966 | A1 | |
| | | | | claims, examples, entire text | | | |
| | | | | JP | 6681493 | B1 | |
| | | | | US | 2022/0023112 | A1 | |
| | | | | US | 2022/0023114 | A1 | |
| | | | | US | 2022/0023115 | A1 | |
| | | | | US | 2022/0023486 | A1 | |
| | | | | US | 2022/0023487 | A1 | |
| | | | | US | 2022/0055014 | A1 | |
| | | | | WO | 2020/122202 | A1 | |
| | | | | WO | 2020/122207 | A1 | |
| | | | | WO | 2020/122209 | A1 | |
| | | | | WO | 2020/122217 | A1 | |
| | | | | WO | 2020/122218 | A1 | |
| | | | | WO | 2020/122219 | A1 | |
| | | | | EP | 3896446 | A1 | |
| | | | | EP | 3895676 | A1 | |
| | | | | EP | 3896095 | A1 | |
| | | | | EP | 3896096 | A1 | |
| | | | | EP | 3896097 | A1 | |
| | | | | EP | 3896118 | A1 | |
| | | | | EP | 3896120 | A1 | |
| | | | | CN | 113196054 | A | |
| | | | | KR | 10-2021-0101248 | A | |
| | | | | CN | 113164304 | A | |
| | | | | CN | 113166307 | A | |
| | | | | CN | 113195555 | A | |
| | | | | CN | 113195598 | A | |
| | | | | CN | 113195599 | A | |
| | | | | CN | 113242865 | A | |
| | | | | KR | 10-2021-0101249 | A | |
| | | | | KR | 10-2021-0101250 | A | |
| | | | | KR | 10-2021-0101251 | A | |

Form PCT/ISA/210 (patent family annex) (January 2015)

INTERNATIONAL SEARCH REPORT
Information on patent family members

International application No.

**PCT/JP2023/010167**

| Patent document cited in search report | Publication date (day/month/year) | Patent family member(s) | | Publication date (day/month/year) |
|---|---|---|---|---|
| | | KR 10-2021-0101252 | A | |
| | | KR 10-2021-0101253 | A | |
| | | KR 10-2021-0101254 | A | |
| WO 2020/184389 A1 | 17 September 2020 | US 2022/0143575 | A1 | |
| | | claims, examples, entire text | | |
| | | EP 3936533 | A1 | |
| | | CN 113544164 | A | |
| | | KR 10-2021-0137068 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020184389 A

- WO 2011086843 A